Europäisches Patentamt

European Patent Office

Office européen des brevets

(11) Publication number: **0 137 975**
**B1**

## (12) EUROPEAN PATENT SPECIFICATION

(45) Date of publication of patent specification: **24.10.90**

(21) Application number: **84109934.4**

(22) Date of filing: **21.08.84**

(51) Int. Cl.⁵: **A 61 B 5/14, A 61 B 10/00**

(54) Blood lancet assembly.

(30) Priority: **15.09.83 US 532423**

(43) Date of publication of application:
**24.04.85 Bulletin 85/17**

(45) Publication of the grant of the patent:
**24.10.90 Bulletin 90/43**

(84) Designated Contracting States:
**BE DE FR GB IT NL SE**

(56) References cited:
**EP-A-0 088 257**
**FR-A-2 508 305**
**GB-A-1 085 141**

(73) Proprietor: **Becton, Dickinson and Company
Mack Centre Drive P.O. Box 2224
Paramus New Jersey 07652-1149 (US)**

(72) Inventor: **Nitzsche, Raymond P.
20 Bennington Drive
Edison New Jersey (US)**
Inventor: **Geiger, Kenneth E.
130 Overlook Avenue Apt. 3F
Hackensack New Jersey (US)**

(74) Representative: **Selting, Günther, Dipl.-Ing. et al
Deichmannhaus am Hauptbahnhof
D-5000 Köln 1 (DE)**

Courier Press, Leamington Spa, England.

## Description

The present invention relates to a blood lancet assembly and more particularly relates to a blood lancet assembly for penetrating living flesh to provide capillary blood for testing.

It is common procedure in hospitals, clinics and doctor's offices to perform tests on blood provided by cutting or puncturing the skin and causing the capillaries of the patient to bleed. The instrument used to cut the capillaries is called a lancet. Lancets are commonly manufactured by stamping and forming the lancet from a sheet metal strip and then sealing it in a flexible package before sterilizing. The lancet typically includes a shank portion which is formed to produce corrugations or ribs, to facilitate holding the lancet, and a planar V-shaped cutting portion. A lancet of this type is shown in U.S. Patent No. 3,046,987 to Ehrlich.

Although efficient for producing capillary blood, the lancet at times causes the user pain and produces more blood than is necessary for some tests. Lay people who have experienced both a bypodermic injection and a lancet puncture for a blood test will usually recite that the lancet is more painful. There are several reasons accounting for the increased pain produced by a lancet. First, a hypodermic needle has ground surfaces that cut and slice through the flesh while the typical planar lancet point has more of a tendency to puncture, stretch and tear the skin surrounding the puncture area. Also, most hypodermic needles are lubricated to reduce pain and insertion forces. Hypodermic needles are usually produced as an assembly with a rigid needle shield which engages the needle hub and protects the needle point from potential damage during shipping storage and final delivery to the user.

One disadvantage to the hypodermic needle is that it has a lumen for the delivery of medication. At the areas where the surface of the lumen intersects the planes of the ground point, there are formed sharp edges that can actually cut pieces of flesh away as the needle penetrates the flesh. This is undesirable in a lancet application since the removal of flesh will probably increase the amount of time needed for the puncture site to heal.

An improved lancet design is described in GB—A—1085141. In this integral lancet and package a retainer section and a cap are integrally molded around the lancet covering all surfaces of the lancet point. The integral retainer and cap contains a reduced cross-sectional area portion at the intersection of the retainer and the cap so that rotation of the cap will sever the cap from the retainer section and allow exposure of the lancet point for use. A sterilization process, according to Higgins, is unnecessary since the elevated temperature of the molten plastic, during the molding process, completely sterilizes the point and adjacent surfaces of the lancet.

The improvement lies in a rigid cover for the lancet point to protect it during handling. Also, when a flexible package containing a sterile lancet is torn or damaged, the lancet may become contaminated before use. The known rigid package would appear to be more resistant to damge before use and therefore, offers improved protection. Also, it is not necessary to pull the lancet out of the package and to risk contamination of the point while doing so.

Nevertheless the integral lancet and package has numerous deficiencies. Most noteworthy is that the cap is molded around the lancet point. With this structure, unless the lancet point is symmetric around the longitudinal axis of the lancet, rotation of the cap relative to the lancet point could cause the lancet point to be deflected or damaged by the plastic cap or cause the lancet to cut through the adjacent cap material. The sharper the lancet cutting edges are, the more fragile they are, and the more likely they will be damaged by cap removal. In addition to apparently precluding the use of very sharp edges on the lancet, the Higgins lancet is not readily lubricated because the point is subsequently placed in contact with molten plastic. Also, the lancet point cannot be inspected after assembly into the retainer section. Therefore, any point damage that occurs during the molding procedure will go unnoticed and a potentially defective product will be transmitted to the user. Further, after using a lancet, it is desirable that the point of the lancet be re-covered in order to prevent contamination and/or infection that may result from someone being accidentally cut with the used lancet point. The fact that material is destroyed in order to remove the cap suggests that the cap may not stay on the lancet tip if it is repositioned thereon. Finally, the exterior surface of the reduced cross-sectional area is not sterile and may contact the user's skin during use. This contact by a non-sterile surface can potentially deposit bacteria on the user's skin adjacent to the point of lancet penetration.

Another lancet assembly as disclosed in EP—A—0 088 257 consists of a handle and a lancet that are integrally formed from plastic. The lancet tip protruding from the handle is embraced by a protective arch which is also an integral part of the assembly. The protective arch offers only some protection against inadvertent piercing of the lancet point but does not give any protection against contamination or infection of the lancet point.

Finally, FR—A—2 508 305 discloses a blood lancet assembly wherein the lancet is movably contained in a housing designed in the form of a fountain-pen. The cap has an internally threaded portion and can be unscrewed from the housing. The housing has an annular collar surrounding the lancet and the lancet protrudes beyond the collar if a releasing element is actuated and the spring pushes the lancet in forward direction. Such a lancet assembly is expensive and its concept is so that the lancet assembly is not a disposable one.

It is the object of the present invention to provide a simple, straightforward, easily fabricated disposable blood lancet assembly which offers a sharp cutting point which may be lubricated and inspected during the assembly process and provides a rigid package to protect the cutting point before use wherein the removal of the rigid package will not damage or compromise the cutting point.

This object is solved, according to the invention, with a lancet assembly having the features of Claim 1.

The blood lancet assembly of the present invention comprises a handle having a distal end and a proximal end to be held by the user. A lancet extends outwardly from the distal end of the handle and terminates in a forward end adapted to cut living animal or human flesh. Also included is a shield having an open end, a closed end and a receptacle therein. This shield is removably engaged with the handle so that the forward end of the lancet is positioned within the receptacle of the shield. The receptacle is larger than the forward end of the lancet so that the lancet is maintained out of contact with the shield.

In accordance with the preferred embodiment of the present invention, a disposable blood lancet assembly comprises a lancet having a rearward end and a forward end shaped to form a point. The lancet point is formed by the intersection of three planes with each of said planes intersecting each other to form three cutting edges. These cutting edges originate at the outside surface of the lancet and terminate at the point. A handle has a proximal end adapted to be held by the user and a distal end connected to the rear end of the lancet so that the forward end of the lancet projects outwardly therefrom. Further, the distal end of the handle includes a raised portion having a side wall. This side wall surrounds the lancet and extends substantially parallel to the longitudinal axis of the lancet. Also provided is a shield having an open end, a closed end and a preferably oblong receptacle therein. This receptacle is longer along its longitudinal axis than the portion of the lancet projecting outwardly from the handle. The shield is held in removable engagement with the handle by an interference fit between the side wall of the raised portion and the receptacle, wherein the forward end of the lancet is positioned within the receptacle. The shield is removable from the handle without touching the lancet and is replaceable on the handle after use of the lancet.

In accordance with the principles of the present invention, a number of advantages and objectives are attained. Primarily, the present invention provides a simple, straightforward, easily fabricated disposable blood lancet assembly. A sharp cutting point is provided which may be lubricated and inspected during the assembly process. In addition the present lancet assembly includes a rigid package to protect the cutting point before use, wherein the removal of the rigid package should not damage or compromise the cutting point.

Fig. 1 is a perspective view of the preferred disposable blood lancet assembly of the present invention;

Fig. 2 is the blood lancet assembly of Fig. 1 illustrating the shield removed and separated from the handle;

Fig. 3 is an enlarged front elevation view of the preferred disposable blood lancet assembly;

Fig. 4 is a cross-sectional view of the blood lancet assembly of Fig. 3 taken along line 4—4;

Fig. 5 is a cross-sectional view of the blood lancet assembly of Fig. 3 taken along line 5—5;

Fig. 6 is an enlarged partial side elevation view of the lancet of the preferred disposable blood lancet assembly;

Fig. 7 is a top plan view of the lancet of Fig. 6;

Fig. 8 is an enlarged partial side elevation view of the lancet of the preferred disposable blood lancet assembly taken 90 degrees from the view of Fig. 6; and

Fig. 9 is a top plan view of the lancet of Fig. 8.

While this invention is satisfied by embodiments in many different forms, there is shown in the drawings and will herein be described in detail preferred embodiments of the invention with the understanding that the present disclosure is to be considered as exemplary of the principles of the invention and is not intended to limit the invention to the embodiments illustrated. The scope of the invention will be measured by the appended claims and their equivalents.

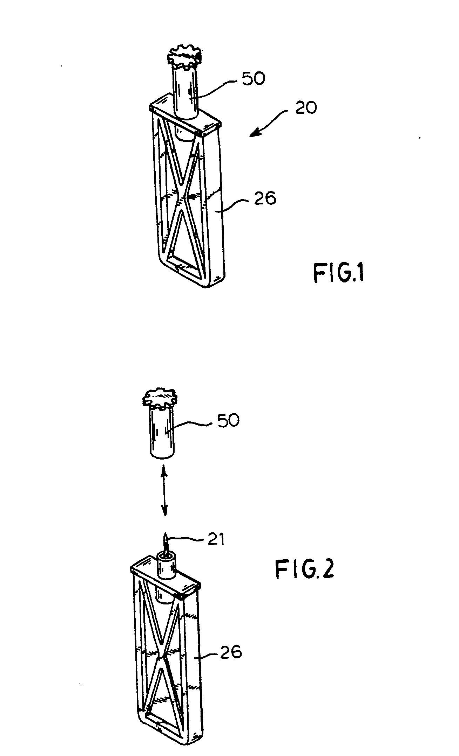

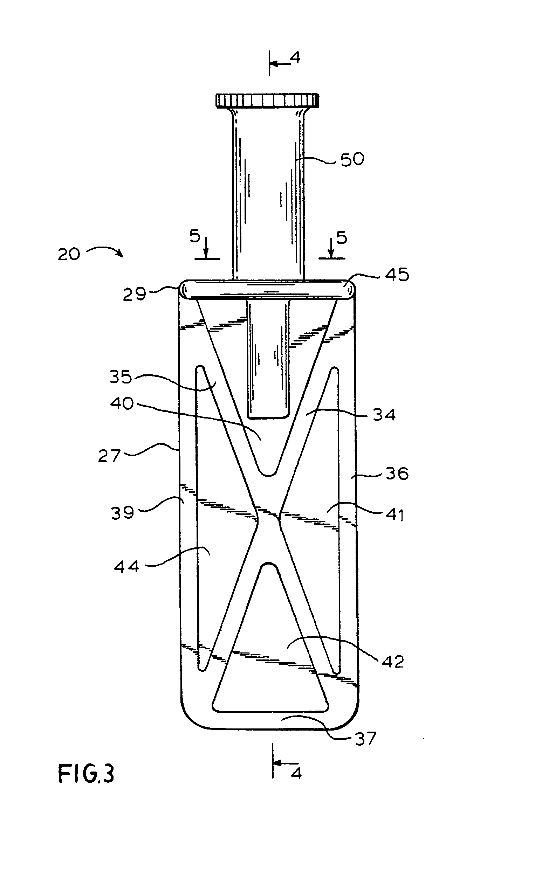

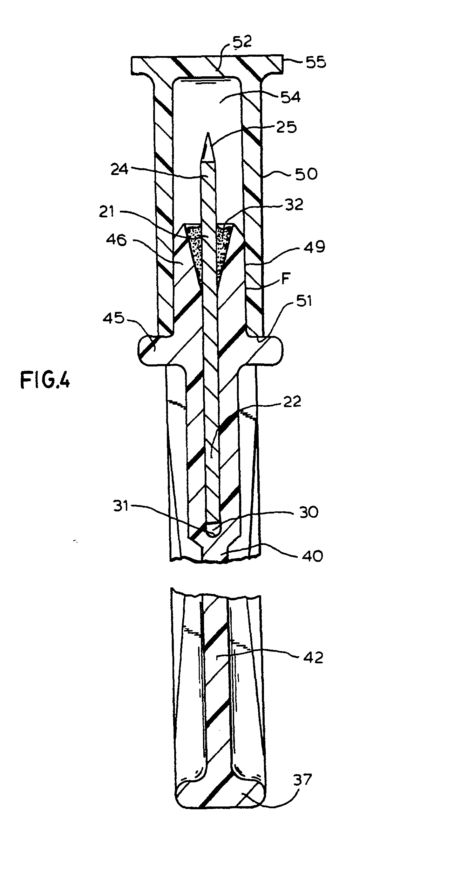

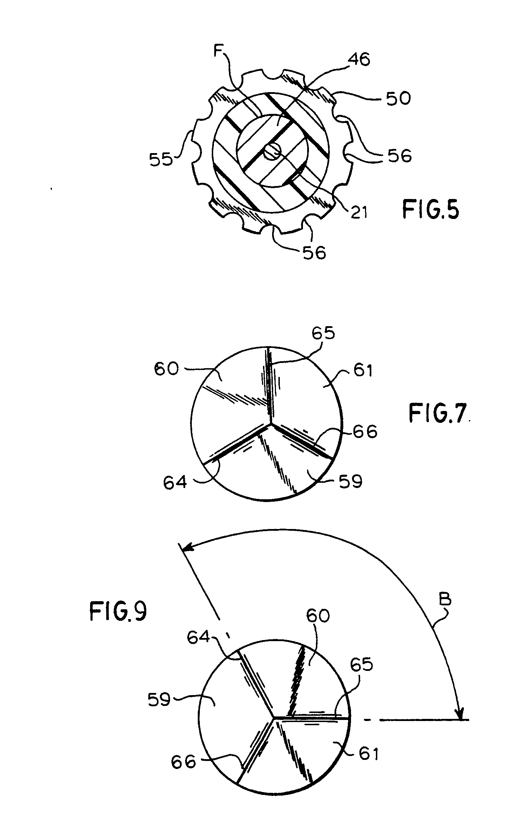

Adverting now to Figs. 1 through 5, a disposable blood lancet assembly 20 includes a cylindrically shaped lancet 21 having a rearward end 22 and a forward end 24 shaped to form a point 25. Also included is a handle 26 having a planar proximal end 27 and distal end 29. Projecting inwardly into the handle from the distal end toward the proximal end is tubular recess 30 terminating at end 31. Lancet 21 is contained within the tubular recess so that its rearward end is adjacent to the end of the tubular recess. The lancet is held in place in the tubular recess preferably by epoxy 32. It will be apparent to one skilled in the art that numerous constructions can be used to join the lancet and the handle and that the arrangement described above is exemplary of these many possibilities. Also, it is within the purview of this invention to include a one-piece lancet and handle assembly.

It should be noted that the distance between point 25 of the lancet and the distal end of raised portion 46 and/or epoxy 32 is the depth which the lancet can penetrate the user's flesh. Accordingly, the desired depth of penetration of the lancet can be controlled by controlling the above-mentioned distance in the design and manufacture of the lancet assembly.

The handle is planar in configuration in order to provide adequate surface areas for the user to grasp the handle between his finger tips and to control the handle which in turn controls the position of the lancet tip. The desirability of this feature will become apparent hereinafter. The planar proximal end of the handle consists preferably of crossed ribs 34 and 35, perimeter ribs 36, 37 and 39, reduced thickness sections 40, 41, 42 and 44, and distal rib 45. The variations in thicknesses between the ribs and the reduced thickness sections provide an improved gripping surface over a smooth surface which may tend to become slippery. This is helpful since the planar portion of the handle in the preferred embodiment may be compact in structure with a typical handle measuring approximately 0.3 inch (7.6 mm) wide, by 0.75 inch (19.1 mm) long by 0.1 inch (2.5 mm) thick at the perimeter ribs. Also, the reduced thickness sections lessen the amount of material required to form the handle and therefore the cost. Projecting outwardly from distal rib 45 is cylindrically shaped raised portion 46 which includes circular side wall 49 which surrounds the lancet and is substantially parallel to the longitudinal axis of the lancet.

Assembly 20 further comprises a shield 50 having an open end 51, a closed end 52 and a receptacle 54 having a circularly shaped cross-section. The shield is positioned over raised portion 46 and is held in removable engagement thereon. By making the outside diameter of raised portion 46 larger than the inside diameter of receptacle 54, an interference fit is created at interface F between the receptacle surface and side wall 49, so that force must be applied to the shield to disengage it from the handle. The interference fit should be tight enough to keep the shield securely engaged to the handle and to protect the lancet from outside contamination without damaging the parts but must be loose enough to allow easy removal of the shield at the time of use. It should be noted that portions of raised portion 46 will touch the user's skin during use of the lancet assembly. However, potential for bacterial contamination of the lancet penetration site, by the raised portion, is reduced because shield 50 covers and protects raised portion 46 from contamination after assembly. To facilitate the removal of the shield from the handle, annular end flange 55 has circumferentially oriented scallops 56.

It should be noted that receptacle 54 is preferably longer along its longitudinal axis than the portion of the lancet projecting outwardly from the handle and that the receptacle has a larger inside diameter than the outside diameter of the lancet. This clearance between the lancet at the shield allows the shield to be removed and replaced without touching and possibly damaging the lancet point.

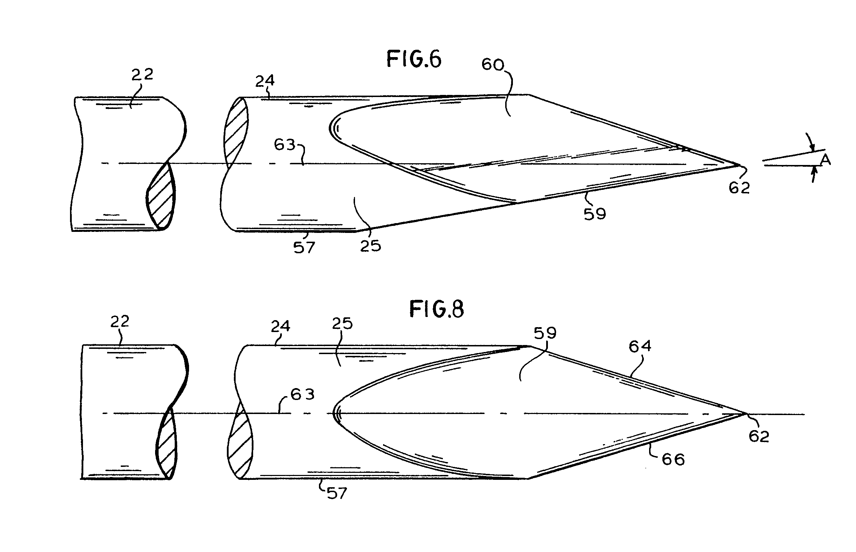

Figs. 6 through 9 depict preferred lancet point 25, as part of the lancet of the present invention. This point is formed by the intersection of planes 59, 60 and 61. The lines of intersection between the planes form cutting edges 64, 65 and 66. Each cutting edge originates at the lancet outside diameter 57 and terminates at leading edge 62 of the point. Preferably, planes 59, 60 and 61 are all at the same angle, angle A, with respect to the longitudinal axis of lancet 21. In the preferred embodiment, angle A is approximately 12°. Also, the planes are oriented so that cutting edges 64, 65 and 66 intersect and form a point having leading edge 62 approximately at longitudinal axis 63 of the lancet. This point, with its leading edge approximately at the longitudinal axis, is called an axial point.

The cutting edges act as sharp flash cutting surfaces which cut as the lancet penetrates the skin. A circular point without cutting edges and a sheet metal lancet without ground edges would have a tendency to puncture, stretch and tear the skin as it penetrates producing what is believed to be a more painful incision. Further, a three cutting edge point makes three cuts which can sever capillaries to produce blood while the well-known planar lancet only makes two cuts. Therefore, a two cutting edge lancet would have to make a larger incision to sever the same area of flesh, and produce the same amount of blood, as the preferred point. Accordingly, the preferred axial point is believed to reduce pain and trauma by producing the same amount of blood with a smaller incision.

Unlike a hypodermic needle, as discussed hereinabove, the preferred lancet does not include a lumen with surfaces which can intersect with the planes of the point to form interior cutting edges which can potentially cut away pieces of flesh during penetration.

The present invention also allows the use of a medical grade silicone lubricant on the lancet point. It is known that this lubricant aids in reducing the forces required for the lancet to penetrate the skin and also the pain perceived by the user. Because shield 50 does not touch the lancet, the lubricant will not be removed or compromised by the act of removing the shield. Along the same lines, the fact that the shield does not touch the lancet allows the edges on the point to be extremely sharp and delicate since they will not be damaged by the removal of the shield.

In the assembly of the instant invention, the shield is not installed until the handle and lancet are assembled. This sequence provides a further advantage in that the point of the lancet may be inspected for any damage that might have occurred during assembly before the shield is installed. Accordingly, a defective product may be eliminated before shipment from the manufacturer.

In use, the user firmly grasps handle 26 with the fingers of one hand and shield 50 in the area of the scallops with the fingers of the other hand. Then the user applies a rotational force to the shield, along with a pulling force, to remove the shield from the raised portion of the handle in order to expose the sharp lancet. The lancet is then quickly thrust toward and into the area of the body where the blood sample is to be taken and promptly removed to allow the blood to flow from the severed capillaries. The shield may then be reinstalled on the handle to cover the lancet and to prevent accidental cuts or infection which may result from inadvertent contact with the exposed lancet. The used lancet assembly is then discarded.

A wide variety of rigid materials is suitable for constructing the handle and shield, however, thermoplastic materials such as polypropylene and polyethylene are preferred. The choice of epoxy formulation is dictated by the materials and processing conditions chosen for handle and lancet. It is perferred that the lancet is made of medical grade stainless steel unless the handle and the lancet are made in one piece. In the latter case, thermoplastic materials such as ABS, polypropylene and polystyrene are preferred. it is also desirable to apply a medical grade lubricant, such as medical grade silicone lubricant, to the portion of the lancet projecting outwardly from the handle. It is preferred that the lancet should be sterile when used. Accordingly, the materials for all components should be selected for compatibility with the sterilization process.

Thus it can be seen that the present invention provides a simple, straightforward, easily fabricated disposable blood lancet assembly which provides a sharp cutting point which may be lubricated and inspected during the assembly process. Also provided is a rigid shield to protect the cutting point, the lubricant thereon and a portion of the structure surrounding the lancet, before use. The present invention further allows the removal of the shield without damaging or compromising the cutting point, and allows the subsequent reinstallation of the shield to protect against contamination and accidental cutting.

## Claims

1. A disposable blood lancet assembly comprising:

a lancet (21) having a rearward end (22) and a forward end (24) with a cutting point (25) adapted to penetrate human flesh;

a handle (26) having a proximal end (27) to be held by the user and a distal end (29) connected with said rearward end (22) of said lancet so that said forward end projects outwardly therefrom; and

a shield (50) covering said forward end (24) and cutting point (25) of said lancet (21) projecting from said handle and having a closed end (52) characterized in that

said shield (50) has an open end (51) and forms a receptacle sized so that said forward end (24) and cutting point (25) are free of direct contact with said shield (50); and

further characterized by means (46, F) for holding said shield (50) in removable engagement on said handle (26) with said forward end (24) and cutting point (25) of said lancet (21) being positioned within said receptacle, said holding means (46, F) allowing removal of said shield (50) from said handle (26) without said lancet (21) touching said shield, said holding means (46, F) further allowing replacement of said shield on said handle after use of said lancet, wherein said holding means includes a raised portion (46) on the distal end (29) of said handle (26) having a side wall (F) surrounding said lancet and being substantially parallel to the longitudinal axis of said lancet, said side wall (F) extending into said receptacle (54) to engage said shield (50) in an interference fit.

2. The disposable blood lancet assembly of Claim 1 wherein said receptacle (54) has a circularly shaped cross section.

3. The disposable blood lancet assembly of Claims 1 and 2 wherein said raised portion (46) on the distal end (29) of said handle (26) has a cylindrical side wall (F).

4. The disposable blood lancet assembly of one of Claims 1—3 wherein said shield (50) includes an outside surface adapted to be held between the finger tips of the user so that said shield may be rotated around its longitudinal axis to facilitate removal from said side wall (F).

5. The disposable blood lancet assembly of one of Claims 1—4 wherein said handle (26) includes a flat portion to be held between the fingers of the user.

6. The disposable blood lancet assembly of Claim 5 wherein said point (25) is formed by the intersection of three planes (59, 60, 61) intersecting each other to form three cutting edges (64, 65, 66), said cutting edges originating at the outside surface of said lancet (21) and terminating at said point.

7. The disposable blood lancet assembly of Claim 6 wherein said lancet (21) is a cylindrically shaped rod with said point (25) formed thereon.

8. The disposable blood lancet assembly of Claim 6 or 7 wherein each of said planes (59, 60, 61) is oriented at the same angle with respect to the longitudinal axis of said lancet (21).

9. The disposable blood lancet assembly of one of Claims 6—8 wherein said planes (59, 60, 61) are oriented so that said point (25) is located approximately at the longitudinal axis of said lancet (21).

10. The disposable blood lancet assembly of one of Claims 1—9 wherein said lancet (21) is made of thermoplastic material or stainless steel.

11. The disposable blood lancet assembly of one of Claims 1—10 wherein said handle (26) and said shield (50) are made of thermoplastic material.

12. The disposable blood lancet assembly of Claim 11 wherein said thermoplastic material is polypropylene or polyethylene.

## Patentansprüche

1. Einweg-Blutlanzetteneinrichtung mit

einer Lanzette (21) mit einem hinteren Ende (22) und einem vorderen Ende (24) mit einer zum Durchdringen von menschlichem Fleisch geeigneten Schneidspitze (25),

einem Griffstück (26) mit einem vom Bediener zu haltenden proximalen Ende (27) und einem distalen Ende (29), das derart mit dem hinteren Ende (22) der Lanzette verbunden ist, daß das vordere Ende von diesem nach außen vorsteht, und

einem das vordere Ende (24) und die Schneidspitze (25) der Lanzette (21) bedeckenden Schutz (50), der von dem Griffstück absteht und ein geschlossenes Ende (52) aufweist, dadurch gekennzeichnet, daß

der Schutz (50) ein offenes Ende (51) aufweist und eine Aufnahme bildet, die derart bemessen ist, daß das vordere Ende (24) und die Schneidspitze (25) ohne direkten Berührungskontakt mit dem Schutz (50) sind, und

ferner gekennzeichnet durch eine Einrichtung (46, F) zum Halten des Schutzes (50) in lösbarem Eingriff an dem Griffstück (26), wobei sich das vordere Ende (24) und die Schneidspitze (25) der Lanzette (21) in der Aufnahme befinden, wobei die Halteinrichtung (46, F) des Entfernen des Schutzes (50) von dem Griffstück (26) ermöglicht, ohne daß die Lanzette (21) den Schutz berührt, und die Halteeinrichtung (46, F) es ferner ermöglicht, den Schutz nach Gebrauch der Lanzette wieder auf das Griffstück aufzusetzen, wobei die Halteeinrichtung einen erhöhten Abschnitt (46) am distalen Ende (29) das Griffstücks (26) aufweist, der mit einer Seitenwand (F) versehen ist, welche die Lanzette umschließt und im wesentlichen parallel zur Längsachse der Lanzette verläuft, wobei sich die Seitenwand (F) in die Aufnahme (54) erstreckt, um mit dem Schutz (50) in Preßsitz zusammenzugreifen.

2. Einweg-Blutlanzetteneinrichtung nach Anspruch 1, bei der die Aufnahme (54) einen kreisförmigen Querschnitt hat.

3. Einweg-Blutlanzetteneinrichtung nach den Ansprüchen 1 und 2, bei welcher der erhöhte Abschnitt (46) am distalen Ende (29) des Griffstrücks (26) eine zylindrische Seitenwand (F) aufweist.

4. Einweg-Blutlanzetteneinrichtung nach einem der Ansprüche 1 bis 3, bei welcher der Schutz (50) eine Außenflache aufweist, die geeignet ist derart zwischen den Fingerspitzen des Benutzers gehalten zu werden, daß der Schutz zur leichteren Entfernung von der Seitenwand (F) um seine Längsachse gedreht werden kann.

5. Einweg-Blutlanzetteneinrichtung nach einem der Ansprüche 1 bis 4, bei welcher das Griffstück (26) einen zwischen den Fingern des Benutzers zu haltenden flachen Bereich aufweist.

6. Einweg-Blutlanzetteneinrichtung nach Anspruch 5, bei der die Spitze (25) durch den Schnittpunkt dreier Ebenen (59, 60, 61) gebildet ist, die sich zur Bildung dreier Schneidkanten (64, 65, 66) schneiden, wobei die Schneidkanten an der Außenfläche der Lanzette (21) entspringen und in der Spitze münden.

7. Einweg-Blutlanzetteneinrichtung nach Anspruch 6, bei der die Lanzette (21) eine zylinderförmige Stange ist, auf der die Spitze ausgebildet ist.

8. Einweg-Blutlanzetteneinrichtung nach Anspruch 6 oder 7, bei der jede der Ebenen (59, 60, 61) im selben winkel bezüglich der Langsachse der Lanzette (21) ausgerichtet ist.

9. Einweg-Blutlanzetteneinrichtung nach einem der Anspruche 6 bis 8, bei der die Ebenen (59, 60, 61) derart ausgerichtet sind, daß die Spitze (25) ungefähr auf der Längsachse der Lanzette (21) liegt.

10. Einweg-Blutlanzetteneinrichtung nach einem der Ansprüche 1 bis 9, bei der die Lanzette (21) aus thermoplastischem Material oder aus Edelstahl hergestellt ist.

11. Einweg-Blutlanzetteneinrichtung nach einem der Ansprüche 1 bis 10, bei der das Griffstück (26) und der Schutz (50) aus thermoplastischem Material hergestellt sind.

12. Einweg-Blutlanzetteneinrichtung nach Anspruch 11, bei der das thermoplastische Material Polypropylen oder Polyathylen ist.

**Revendications**

1. Un appareil de prélèvement sanguin par lancette, à usage unique, comprenant:

une lancette (21) pourvue d'une extrémité arrière (22) et d'une extrémité avant (24) munie d'une pointe acérée (25) agencée pour perforer la chair humaine,

une poignée (26) munie d'une extrémité proximale (27) maintenue par l'utilisateur et d'une extrémité distale (29) reliée à ladite extrémité arrière (22) de ladite lancette, de manière que ladite extrémité avant fasse saillie vers l'extérieur à partir de celle-ci, et

un capuchon protecteur (50) recouvrant ladite extrémité avant (24) et la pointe acérée (25) de ladite lancette (21) faisant saillie à partir de ladite poignée et comprenant une extrémité fermée (52), caractérisé en ce que ledit capuchon protecteur (50) comprend une extrémité ouverte (51) et forme un réceptacle ou cavité, dimensionné de manière que ladite extrémité avant (24) et la pointe acérée (25) ne sont pas en contact direct avec ledit capuchon protecteur (50), et caractérisé en outre par des moyens (46, F) pour maintenir ledit capuchon protecteur (50) engagé de manière amovible sur ladite poignée (26), dans lesquels ladite extrémité avant (24) et la pointe acérée (25) de ladite lancette (21) sont positionnées dans ledit réceptacle, lesdits moyens de maintien (46, F) permettant l'enlévement dudit capuchon protecteur (50) de ladite poignée (26) sans que ladite lancette (21) entre en contact avec ledit capuchon protecteur, lesdits moyens de maintien (46, F) permettant en outre la remise en place dudit capuchon protecteur sur ladite poignée après l'utilisation de ladite lancette, de manière que lesdits moyens de maintien comportent une partie surélevée (46) à l'extrémité distale (29) de ladite poignée (26), comprenant une paroi latérale (F) entourant ladite lancette et étant sensiblement parallèle à l'axe longitudinal de ladite lancette, ladite paroi latérale (F) s'étendant dans ledit réceptacle (54) pour venir en prise avec ledit capuchon protecteur (50) par un ajustement serré.

2. L'appareil de prélèvement sanguin par lancette à usage unique, selon la revendication 1, dans lequel ledit réceptacle (54) est de forme circulaire en coupe transversale.

3. L'appareil de prélèvement sanguin par lancette à usage unique, selon les revendications 1 et 2, dans lequel la partie surélevée (46) de l'extrémité distale (29) de ladite poignée (26) comprend une paroi latérale cylindrique (F).

4. L'appareil de prélèvement sanguin par lancette à usage unique, selon l'une quelconque des revendications 1 à 3, dans lequel ledit capuchon protecteur (50) comprend une surface extérieure adaptée pour être maintenue entre la bout des doigts de l'utilisateur, de manière que ledit capuchon puisse être tourné autour de son axe longitudinal pour faciliter l'enlèvement de ladite paroi latérale (F).

5. L'appareil de prélèvement sanguin par lancette à usage unique, selon l'une quelconque des revendications 1 à 4, dans lequel ladite poignée (26) comprend une partie plane devant être maintenue entre les doigts de l'utilisateur.

6. L'appareil de prélèvement sanguin par lancette à usage unique, selon la revendication 5, dans lequel ladite pointe (25) est formée par l'intersection de trois plans (59, 60, 61), s'intersectant mutuellement pour former trois bords coupants (64, 65, 66), lesdits bords coupants ayant pour origine la surface extérieure de ladite lancette (21) et se terminant à ladite pointe.

7. L'appareil de prélèvement sanguin par lancette à usage unique, selon la revendication 6, dans lequel ladite lancette (21) est en forme de tige cylindrique, munie de ladite pointe ménagée sur celle-ci.

8. L'appareil de prélèvement sanguin par lancette à usage unique, selon la revendication 6 ou 7, dans lequel chacun desdits plans (59, 60, 61) est orienté selon le même angle par rapport à l'axe longitudinal de ladite lancette (21).

9. L'appareil de prélèvement sanguin par lancette à usage unique, selon l'une quelconque des revendications 6 à 8, dans lequel lesdits plans (59, 60, 61) sont orientés de manière que ladite pointe (25) est située approximativement sur l'axe longitudinal de ladite lancette (21).

10. L'appareil de prélèvement sanguin par lancette à usage unique, selon l'une quelconque des revendications 1 à 9, dans lequel ladite lancette (21) est réalisée en matériau thermoplastique ou en acier inoxydable.

11. L'appareil de prélèvement sanguin par lancette à usage unique, selon l'une quelconque des revendications 1 à 10, dans lequel ladite poignée (26) et ledit embout (50) sont réalisés en matériau thermoplastique.

12. L'appareil de prélèvement sanguin par lancette à usage unique, selon la revendication 11, dans lequel ledit matériau thermoplastique est du polypropylène ou du polyéthylène.

FIG.1

FIG.2

FIG.3

FIG.4

FIG.5

FIG.7.

FIG.9

FIG.6

FIG.8

EP 0 137 975 B1